(19) **Europäisches Patentamt**

**European Patent Office**

**Office européen des brevets**

(11) **EP 0 653 062 B1**

(12) **EUROPEAN PATENT SPECIFICATION**

(45) Date of publication and mention
of the grant of the patent:
**02.05.2003 Bulletin 2003/18**

(21) Application number: **93915369.8**

(22) Date of filing: **15.06.1993**

(51) Int Cl.7: **G01N 33/48**, A61M 1/36

(86) International application number:
**PCT/US93/05735**

(87) International publication number:
**WO 94/027698 (08.12.1994 Gazette 1994/27)**

(54) **CONTINUOUS CENTRIFUGATION PROCESS FOR THE SEPARATION OF BIOLOGIC COMPONENTS FROM HETEROGENEOUS CELL POPULATIONS**

KONTINUIERLICHES ZENTRIFUGATIONSVERFAHREN ZUM ABTRENNEN VON BIOLOGISCHEN KOMPONENTEN AUS HETEROGENEN ZELLPOPULATIONEN

PROCEDE DE CENTRIFUGATION CONTINUE DESTINE A LA SEPARATION DE CONSTITUANTS BIOLOGIQUES DE POPULATIONS DE CELLULES HETEROGENES

(84) Designated Contracting States:
**BE DE FR GB IT**

(30) Priority: **28.05.1993 US 69077**

(43) Date of publication of application:
**17.05.1995 Bulletin 1995/20**

(73) Proprietor: **BAXTER INTERNATIONAL INC.
Deerfield, IL 60015 (US)**

(72) Inventors:
• **LAKE, William, C.
Laguna Niguel, CA 92656 (US)**
• **GIESLER, Richard
Deerfield, IL 60015 (US)**
• **VAN EPPS, Dennis
Cary, IL 60013 (US)**
• **CHAPMAN, John, R.
Lake Villa, IL 60046 (US)**

• **MARTINSON, Jeffrey, A.
Mundelein, IL 60060 (US)**
• **ELLIS, Dale, R.
Wonder Lake, IL 60097 (US)**
• **AONO, Frederick
Arlington Heights, IL 60004 (US)**
• **BISCHOF, Daniel, F.
McHenry, IL 60050 (US)**

(74) Representative: **MacGregor, Gordon et al
Eric Potter Clarkson,
Park View House,
58 The Ropewalk
Nottingham NG1 5DD (GB)**

(56) References cited:
**US-A- 4 146 172        US-A- 4 722 790
US-A- 4 783 336        US-A- 4 927 749
US-A- 4 935 147**

## Description

TECHNICAL FIELD

The present invention concerns a system for separating a specific cell population from a heterogeneous cell mixture. An embodiment of the invention concerns a closed, sterile continuous flow process for separating a nucleated heterogeneous cell population from a large volume of heterogeneous cell mixture in a relatively short time.

BACKGROUND OF THE INVENTION

In the field of cell separation, it is common to separate cells from plasma in blood and also to separate by centrifugation various types of cells such as red cells from white cells, and the like. Centrifugation segregates cells according to their differing specific gravities. However, there is often a need to separate from a suspension cells having specific gravity only slightly different from those of other cells in the suspension. If the cells are of nearly equal specific gravity, they may not be separated by centrifugation.

For example, it may be desirable to isolate various types of leukocytes from a bone marrow concentrate or a peripheral blood cell concentrate. Or, it may be desirable to perform selective separation of tumor cells from a bone marrow concentrate, for example, hematopoietic progenitor cells. It may be desirable to selectively separate specific T-lymphocyte subset populations (helper-inducer or suppressor-cytotoxic T-lymphocytes) from a lymphocyte concentrate that is prepared using a blood cell separator.

Additionally, it may be desirable to selectively separate precursors of lymphokine activated killer (LAK) cells, tumor infiltration lymphocyte (TIL) cells, or activated killer monocytes, from lymphocyte or monocyte cell concentrates or from a tissue cell preparation.

By current techniques of the prior art, such as Sauer, et al, U.S. Patent No. 4,710,472, magnetic separations in significant quantities of individual subsets of cells from larger populations became possible. This, in turn, opens up new vistas of research and therapeutic techniques, making use of the purified cell populations that may be obtained.

Another current practice in the field of cell separation, utilizes sheet membranes, hollow fibers, or packed beds of either beads or particles having physically adsorbed or covalently attached chemicals or biochemicals, such as antibodies. By these means certain populations of cells are selectively separated from whole blood, blood components, bone marrow, tissue digests, or other types of cellular suspensions. These devices are designed to allow continuous inflow and return of the cell mixtures. When used to process blood, these devices usually operate at the normal rates of blood flow and under conditions in which the concentration of desired cells can be very low compared with other cell types. The separation process, therefore, is often not efficient.

Immunoaffinity cell separation systems for blood and bone marrow conventionally require two separation processes: an initial cell separation to remove red blood cells and the immunoaffinity cell separation to capture or deplete a specific "target" cell population, such as a nucleated heterogeneous cell population. In the immunoaffinity separation step, a biological particle such as an animal erythrocyte, is modified by coupling to its surface a monoclonal or polyclonal antibody or other biological selected to specifically bind to an antigen or immunogenic marker on the surface of the target cell. A high density particle/target cell conjugate, such as an erythrocyte rosette, is thereby created. Because a significant incubation time is required for particle/cell bonding to occur in such systems, the cell mixture is usually centrifuged twice, once to promote binding of the particle-antibody conjugate to the target cell and a second time to separate the particle/target cell conjugate using a high density separation media so that only the high density erythrocytes and erythrocyte/target cell conjugates will sediment through the medium. Separation is thus effected with efficiencies of up to 95%.

In addition to the many steps required to effect immunoaffinity separations using these techniques, the immunoaffinity cell separator systems currently described in the literature are limited in the volume of cell preparations that can be processed, and none can be performed in a closed, continuous flow on-line procedure with a patient.

In view of these difficulties, the need exists for new and improved methods of continuously separating a specific cell population from a heterogeneous cell mixture, especially for separating from a cell mixture populations of cells that differ in specific gravity and/or sedimentation velocity only slightly from other cells in the mixture.

US-A-4722790 discloses the use of beads coated with a binder, which can bind specific cells in a cell mixture. The beads are contained in a bag, to which a cell mixture is added and the bag is then rotated to improve binding of the specific cells and produce a bead containing phase and a specific cell depleted phase. The latter phase is then removed from the bag, leaving the bead containing phase behind.

The precharacterising part of Claim 1 is based on this disclosure and the distinguishing features of the invention are set out in the characterising part of the claim.

The invention provides the advantage that large volumes of cells can be centrifuged in a closed, sterile, continuous flow process with a high degree of selectivity provided by immunoaffinity cell separation. The invention is especially useful for separating a target cell population from a heterogeneous cell suspension in which the density and/or sedimentation velocity of the target cells is insufficiently differentiated from those of other cells in the suspension to effect separation by centrifugation with or without the use of high density separation media.

The method may be used for removing from heterogeneous cell populations--such as blood, blood components, blood substitutes, bone marrow, and tissue digests--biologic components including the following: hematopoietic cells, including all leukocyte subpopulations and pluripotent stem cells; tumor cells; tissue culture cell lines, including hybridoma cells; antigen specific lymphocytes; infectious agents, including bacteria, virus and protozoa; and toxic substances, including but not limited to drugs or pharmaceuticals and animal, microbial and plant toxins. The method can be used for therapeutic and diagnostic applications and can be utilized to perform both positive and negative cell selections. In positive cell selection, the bonds between the captured cells and the particles are released and the isolated captured cells are the products used in therapeutic or diagnostic applications. In negative cell selection, the cell mixture depleted of the captured cells (i.e., the "target cells") is the cell product.

Like known affinity cell separation procedures, the present method may use separation particles with a specific affinity for the target cells or having chemically attached thereto a biological molecule with a specific affinity for the target cells. In one continuous flow process for conducting leukapheresis, the affinity particles are continuously fed at a predetermined ratio to the cell mixture through a mixing chamber wherein the particle/target cell conjugates are formed. From the mixing chamber the entire cell mixture, containing the particle/target cell conjugates, passes into a continuous flow centrifuge. Any of a number of commercial continuous flow centrifuges and elutriators that employ disposable plastic inserts including chamber means for facilitating density based separation can be used, such as the "Fenwal Models CS 3000" and "Autopheresis C" sold by Baxter International Inc, of Deerfield, Illinois; "IBM Model 2997" sold by Cobe manufacturing of Lakewood, Colorado; and "Beckman J-Series Elutriation Centrifuges" sold by Beckman Instruments, Palo Alto, California.

In the "Fenwal Autopheresis C System", anticoagulated whole blood may be pumped into a separation device, where plasma is initially separated in a centrifugal density separation chamber. From there, the separated plasma is filtered through a rotating membrane filter and directed into a collection chamber. Concentrated cellular components are pumped from the density separation device to an in-line reinfusion reservoir. Undesired cellular components are returned to the donor, typically through the same needle.

The "Fenwal CS 3000" Blood Cell Separator employs a two-stage, centrifugal density separation and collection process. In a typical platelet collection, a depletion procedure using the CS3000, whole blood is withdrawn from a donor or a blood reservoir and pumped into a separation chamber, where the less dense components, (e.g. platelets and plasma) are separated from the more dense components (e.g. red blood cells). The platelet-containing plasma is transferred from the first chamber into a second chamber via one outlet port, while the red blood cells are removed from the first chamber via a second outlet port. The platelets are separated from the plasma in the second chamber by centrifugal force, and the platelet-deficient plasma is then removed, leaving platelet concentrate in the second chamber.

The model 2997 uses a generally belt-shaped disposable chamber mounted within a rotor in the centrifuge housing. In a typical procedure, whole blood is directed into the belt and, under centrifugal force, is separated into lighter and heavier components as the blood flows circumferentially through the belt. Depending on the particular configuration of the belt, and the location of pick-off points within the belt, the blood may be separated into desired components which are withdrawn from the belt. Other components may be returned to a donor or to a reservoir from which the whole blood is initially drawn.

Commercial sterile plastic inserts having integral chambers, which may be used as mixing and separation chambers , can be purchased for use with each of these machines. For instance, for use with the "Fenwal CS 3000", there are available the "Fenwal" Disposables Nos. 4R2230 and 4R2210. As described in U.S. Patent 4,526,515, for example, these disposables contain a first receptacle useful as a separation chamber, and a second receptacle useful as a collection bag. Typically the commercial plastic disposable inserts can be purchased with or without preattached saline, anticoagulant supplies, and apheresis needles for use in continuous processing and return of blood to a patient.

In a preferred embodiment, the cell mixture and affinity particles are introduced in continuous flow directly into a chamber of the centrifuge which acts as a mixing chamber either as separate streams or mixed as a single stream. In the mixing chamber within the centrifuge shear forces are controlled so that formation of stable bonds between the particles and the target cells is enhanced.

It has been unexpectedly found that the centrifugal force within the rotating chamber also acts to substantially enhance intimate contact between the particles and the target cells, overcoming the adverse effects of shear forces created by rotation, so that the bonding reaction forming the particle/cell conjugate occurs readily, instantaneously in some cases, within the centrifuge. For this reason the time needed for incubation of the particles is eliminated. Therefore, the particles and heterogeneous cell mixture can be fed into and removed from the centrifuge at the continuous flow rate that would normally be used to separate any component from the heterogeneous cell mixture without substantially increasing the residence time in the centrifuge to allow an "incubation" period for formation of the particle/cell complex.

In some cases, for instances in separation of a leukocyte target cell from whole blood, it is preferred to perform a preliminary centrifugation step without the use of particles. In this preliminary step, those cell populations naturally characterized by a density different than that of others in the cell mixture can be removed before the immunoaffinity separation is undertaken. For instance, with whole blood, an initial centrifugation step can be used to separate the red blood cell population from the leukocytes. Then, in a second step the leukocyte mixture can be treated as the heterogeneous cell mixture used in the continuous centrifugation immunoaffinity separation method.

In other cases, such as the separation of stem cells from a heterogeneous cell mixture, the concentration of the target cell is too limited to use the preliminary centrifugation step, which would fail to capture a significant fraction of the target cells in the concentrate. Greater efficiency of target cell separation can be achieved in this case by utilizing a single step continuous centrifugation immunoaffinity separation.

Particles used for continuous centrifugation immunoaffinity separation are selected and/or designed not only to bind to the target cell population with great specificity, but also to sufficiently alter the sedimentation velocity of the particle/cell conjugate during centrifugation so that continuous separation by centrifugation is possible. The particle selection process is described in detail on pages 18-20, infra. In the centrifuge, the particle/target cell conjugates are directly separated from the other components in the cell mixture by the operation of centrifugal force and their altered sedimentation velocities, with or without the use of a density gradient medium. The decision whether to employ a density gradient medium will depend upon how different the sedimentation velocity of the particle/cell conjugate is from that of other cell populations in the cell mixture as determined by means well known in the art.

The remainder of the cell mixture can either be discarded or returned to the patient, as desired. Depending upon the type of commercial separator machine used, continuous reinfusion to the patient can proceed simultaneously with the continuous separation method herein. It is the particular advantage of the continuous centrifugation immunodensity separation method taught in this invention that large volumes of cell mixture can be processed in a closed, continuous flow on-line procedure with a patient while all blood components not captured by the particles are returned to the patient without any risk of contamination.

As a protective measure, a particle capture device preferably is employed downstream of the centrifugal cell separator to remove any residual particle/target cell conjugates and/or particles from the remainder of the cell mixture before it is returned to the patient. The particle capture device, usually either a filter or a magnetic device (if the particles used contain magnetic or ferromagnetic materials) is typically located along the downstream portion of the integral, disposable plastic insert used in the centrifugation step. If the capture device is a magnet, the downstream portion of the plastic tubing inset is provided with means for passing the remainder of the cell mixture in close proximity to the magnet so that any remaining particles are retained in a fixed location as remaining, unbound portions of the cell mixture are removed from the location.

If desired, the particle/target cell conjugates recovered from the centrifugal cell separator can be processed to release the particles from the target cells using known methods. For instance, a chemical process, such as reducing a disulfide bond linkage, an enzymatic process, such as proteolytic treatment with a clinical grade preparation of chymopapain, i.e. Discase, or with a growth factor like interleukin 2 or hematopoietic growth factors can be used to expand and release target cell populations from particles. Alternatively, a competitive process such as free antigen or ligand or a physical process such as dissolving the particle from the target cell, or physically removing it by shear forces or energy transfer are contemplated. If the particles are recovered intact, they can be recycled and reused, if desired.

The principal of separation employed in the new technology is the selective alteration of a target population's sedimentation velocity. The sedimentation of cells can be described by Stoke's equation for the settling of a sphere in a gravitational field:

$$V = \frac{d^2 \, (os - oL) \times g}{18 \, N}$$

where V = sedimentation rate or velocity of the sphere; d = diameter of the sphere; os = sphere density; oL = liquid density; N - viscosity of the liquid medium; and g = gravitational force. From Stoke's equation, it can be seen that the rate of sphere sedimentation is proportional to the size of the sphere; the sedimentation rate is proportional to the difference in density between the sphere and the liquid; the sedimentation rate is zero when the sphere density is the same as the liquid density; the sedimentation rate decreases as the liquid viscosity increases; and the sedimentation rate increases as the gravitational force increases.

In applied cell separation the sphere represents the target cell population. The binding of particles to the target cell increases its effective diameter, thereby altering its sedimentation velocity. An additional change to the sedimentation velocity can be accomplished by selecting particles that are either more or less dense than the target cells. In this way the sedimentation velocity of the target cell can be made to be greater or less than that of non-target cells. Moreover, the efficiency of cell separation can be altered by selecting different g forces (i.e., by altering the speed of the rotor and/or varying the radius of the rotor in the separation chamber), different liquid medium density, and different times of exposure to the g force i.e., by adjusting the flow rate of the cell suspension containing the conjugates through the sedimentation chamber.

BRIEF DESCRIPTION OF THE DRAWINGS

The invention will be better understood and appreciated by carefully studying the following detailed description of a presently preferred exemplary embodiment of this invention when taken in conjunction with the accompanying drawings, of which:

Figure 1 is a schematic diagram of a portion of an exemplary continuous flow centrifugation system having a mixing chamber located outside of the centrifuge.

Figure 2 is a schematic diagram of a portion of an exemplary continuous flow centrifugation system wherein the immunoaffinity particles are metered into a stream of the mixture to be separated and passed into a container located within the centrifuge for binding to the target particle therein.

DETAILED DESCRIPTION OF THE PREFERRED EMBODIMENT

In this invention, a method is provided for on-line, continuous flow selective separation of a specific target cell population from a heterogeneous cell mixture having at least one extraneous cell population with a density and/or sedimentation velocity too close to that of the target cell population to efficiently use known methods of continuous flow centrifugation separation. As a first step, the heterogeneous cell mixture can optionally be subjected to a means, such as a continuous flow centrifuge, for separating a selective cell concentrate based upon the physical properties of the concentrate, thus yielding from the heterogeneous cell mixture a selective cell concentrate. Usually all cell populations in the selective cell concentrate, including the target cell population, will have similar sedimentation velocities. As described above, the initial separation can be effected using any of a number of known and/or commercially available on-line, continuous flow centrifuges for processing large volumes of heterogeneous cell populations. The selective cell concentrate thus obtained is then preferably used as the feed in the method of continuous centrifugation immunoaffinity separation described herein.

The particles have chemically attached thereto, preferably by means of a covalent or a high affinity bond, a biologic substance capable of binding only the desired cells to the exclusion of other cells. Examples of such bonded substances include antibodies, antigens, proteins generating immune responses, nucleotides, glycoproteins, polysaccharides, lipopolysaccharides, and hormones.

Descriptions of such binding effects are found in numerous publications. T cells and B cells taken from patients with systemic lupus and a variety of other rheumatologic diseases where anti-DNA antibodies are present can bind DNA, Bankhurst, A.D. and Williams, R.C. Jr., "Identification of DNA-binding Lymphocytes in Patients With Systemic Lupus Erythematosus", Journal of Clinical investigation, 56: 1378-1385 (1975). This property is specific to those cells which recognize DNA as an antigen, and is the case with a variety of antigens where the antigen specific T cells and B cells carry receptors for those antigens.

It has also been demonstrated that certain hematopoietic progenitor cells bind specific sugars preferentially, Aizaw, S. and Tavassoli, M.," Molecular Basis of the Recognition of Intravenously Transplanted Hemopoietic Cells by Bone Marrow," Proceedings of the National Academy of Sciences 85: 3180-83 (1988).

Additionally, it has recently been shown that a series of proteins known as "selectins", which also bind sugars, have been discovered in certain leukocytes, Lasky L.A. "Selectins: Interpreters of Cell-Specific Carbohydrate Information During Inflammation", Science 258: 964-969 (1992). The selectins mediate the selective adherence of these particular leukocytes to blood vessel walls.

This binding phenomenon has also been demonstrated for hormones. Specifically, human growth hormone, a peptide hormone, has successfully been bound to human peripheral lymphocytes, further demonstrating potential uses for the present invention, W. Kiess and O. Butenandt, "Effect of Enzyme and Enzyme Inhibitors on Specific Binding of HGH to Human Peripheral Lymphocytes", Acta Endocrinologica 109: 139-144 (1985); Smal, Jean et al. "Receptor-Binding and Down-Regulatory Properties of 22,000-MW Human Growth Hormone and Its Natural 20,000-MW Variant on Im-9 Human Lymphocytes", Journal of Biochemistry 225, 283-289 (1985); Eshet, R., Peleg, S. and Laron, Z. "Direct Visualization of Binding, Aggregation and Internalization of Human Growth Hormone in Cultured Human Lymphocytes", Acta Endocrinologica 107: 9-15 (1984).

Finally, this binding phenomenon can also be found in the case of lipopolysaccharides (endotoxins). The CD14 antigen found selectively on blood monocytes has been shown to be a receptor for endotoxin.

This binding technique can also be used with any proteins against which the host cell generates an immune response. B cells or T cells specifically reactive to those antigens can be selected out from the heterogeneous mixture. This includes a broad spectrum of protein, nucleic acid, and carbohydrate antigens.

Additionally, any protein having a specific cell surface receptor could potentially be used to selectively remove or harvest reactive cells from a complex mixture of cell types. These include, for example, IgG where specific receptors for this antibody exist on monocytes, neutrophils, B cells, and T cells, Titus, J.A., Sharrow, S. O., and Sagal, D.M. "Analysis of Fc (IgG) Receptors on Human Peripheral Blood Leukocytes by Dual Fluorescence Flow Microfluoremetry", Journal of Immunology 130: 1152-1158 (1983).

The binding process could also be used with cytokines such as interleukin 2 or interleukin 1, where specific receptors have been identified on T cells, smith, W.B., Gamble, J.R. and Vadas, M.A., "Cytokines in the Inflammatory Responses", Interferons and Cytokines 21: 26-29 (1992).

The present invention is also applicable for growth factors such as stem cell factor (C-kit ligand), etc., where cell surface receptors have been identified on hematopoietic progenitors, Papayannapoulou, T., Brice, M., Broudy, V.C., and Zsaebo, K.M., "Isolation of C-kit Receptor-Expressing Cells from Bone Marrow, Peripheral Blood, and Fetal Liver: Functional Properties and Composite Antigenic Profile", Blood 78: 1403-1412 (1991).

The criteria for use of a given component to bind to cells and allow for differential selection and separation of these from a mixture of cells is that the affinity or avidity of the cell binding to the particle is in excess of the forces acting against the maintenance of this complex. This includes such forces as cell boyency, shear force, etc. Thus, any cell particle complex that can be maintained during centrifugation through a ligand-receptor interaction is a candidate for this continuous flow process of cell selection.

The heterogeneous cell mixture, which may comprise a cell concentrate obtained as above described, is intimately contacted with the particles having a chemically attached biologic substance, bound by either a covalent or a strong ionic bond, to enhance formation of the particle/cell conjugate by binding of the biologic substance to a receptor site on the target cell. Formation of a cell concentrate is preferred because the target cell population to be separated is present at higher concentration than in the original cell mixture. A higher concentration of target cell population tends to favor separation kinetics because numerous unwanted cell types can usually be greatly reduced in number by the preliminary, typically centrifugal cell separation process, thereby greatly reducing non-specific cell reactions. For example, the collection of a lymphocyte cell concentration with minimal red blood cell, platelet, and granulocyte contamination may be effected using a blood cell separator. The separation times in the subsequent centrifugation step are usually reduced and the number of cell types in the concentrate is fewer so that the final product has fewer undesirable contaminating cells.

In one embodiment , the cell mixture or concentrate and the particles are introduced together into a mixing chamber located within the centrifuge so that intimate contact of the particles is substantially enhanced by the action of centrifugal forces thereupon. The particles are introduced as a stream fed slowly or metered into any conduit or passage through which the cell mixture or concentrate is flowing. It has been discovered that the time required for incubation can be so reduced that formation of the conjugate occurs virtually upon contact. For this reason, the particles and the cell mixture or concentrate can be continuously fed into the mixing chamber within the centrifuge at the desired centrifugation flow rate.

Using one variation of the latter method, therefore, confers the considerable advantage that blood, or other bodily fluid, from a patient can be continuously withdrawn at any safe and customary rate by means of an apheresis needle attached to the disposable plastic centrifuge insert, and transferred directly into a mixing chamber within the centrifuge along with the particle means for formation of the conjugate. Then the conjugate can be transferred sequentially through the separation and collection chambers at substantially the same flow rate as it was withdrawn from the patient for separation and collection of the particle/cell conjugate. Meanwhile the remainder of the patient's blood can be reinfused as a final step in the continuous process via a second apheresis needle attached to the disposable plastic inset.

Since the particle/cell conjugates will have significantly different sedimentation velocity than those of the remainder of the cells, the particle/cell conjugates can be readily separated from the other cells during centrifugation. However, when it is desirable to ensure that all unbound and bound particles have been removed from the cell mixture--for instance when blood is being continuously processed on-line and reinfused into a patient--one may use magnetic or paramagnetic particles and effect a secondary separation step by routing the return flow of the cell mixture in close proximity past a magnetic means. Alternatively, if the particles used are of a size suitable for capture by a filter, the return flow can be passed through a suitable filter to capture residual particles and particle/cell complexes. In the secondary separation step any remaining particles are held stationary by the magnet or filter while the remainder of the cell mixture flows onward and ultimately returns to the patient unimpeded.

After separation of the particle/cell conjugate from other cells, as described above, the target cell population can be freed from the particles or vice versa, for example by eliminating the bond between the particles and the cells in a known manner, so that a purified, selected population of cells may be provided for further use. For example, target cells may be cleaved using an enzymatic reaction, a reducing agent in the case of a disulfide bond, or a competitive inhibition reaction between the desired protein and the target cell surface. Alternatively, the unbound cells may be the desired cells, being removed from the particle/cell conjugates.

It is also preferred for the container which contains the particles to be aseptically connected to a flexible, multiple-chamber insert for the blood cell separation centrifuge, so that freshly collected blood cells, or other bodily fluids, can be aseptically combined in the mixing chamber, without any need of forming a sterile connection there between. This greatly simplifies the use in accordance with this invention, and also increases the likelihood that there is no breach of aseptic conditions.

Further , one method by which the invention can be practiced includes the following steps. Blood from the patient is either collected in a first container or the patient can be connected to a blood separation centrifuge, the centrifuge being operated to form a cell concentrate which is collected in the first container. The first container is usually sealed until separation of the target cell population is desired. If the particles are not already in the container, they can be placed in the container in some aseptic manner before the separation step. It is usually difficult or impossible to determine the number of target cells in a heterogeneous cell mixture. However, the number of nucleated cells can more readily be obtained by known means. Therefore, sufficient particle means are used to create a ratio of particles to nucleated cell population in the range from about 1:1000 to 1000:1, and more preferably from about 1:100 to 100:1. If desired, the particles can be sealed into an inner container positioned within the first container so that the inner container can be broken from outside of the first container to cause release of the particles into the first container.

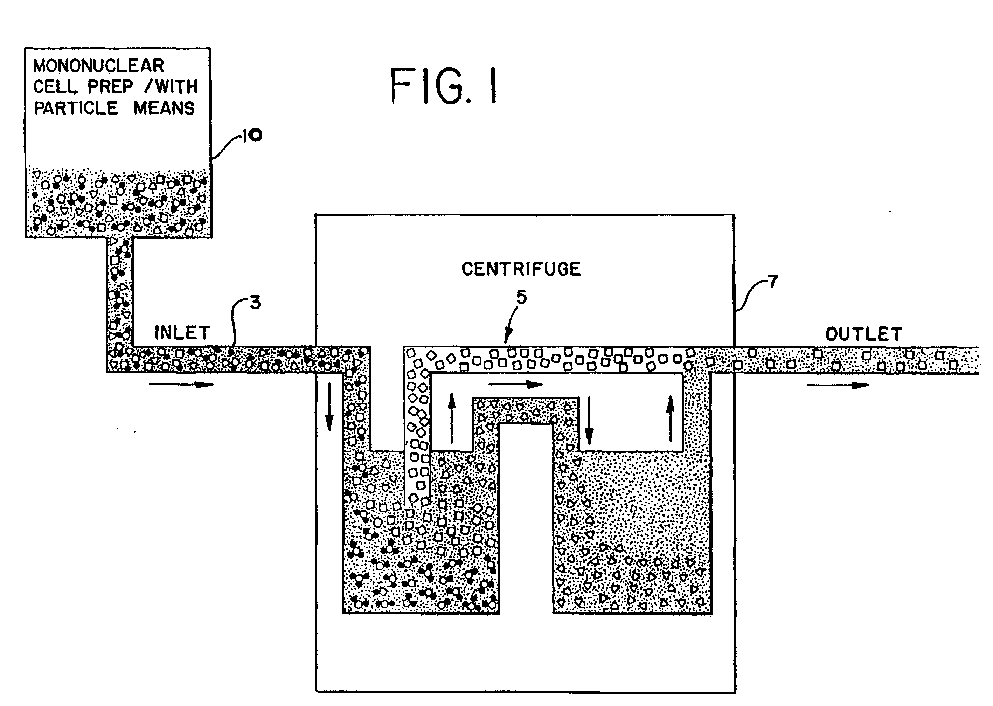

More commonly, as illustrated in Figure 1, the cells and the particles are introduced into a first container 10 used as a mixing chamber where they are mixed by any known means (not shown) . Then, the mixing chamber 10 is connected, if not already integrally connected, to the inlet 3 of a clamped disposable separation insert 5 to a continuous flow centrifuge 7 and the disposable insert is placed into the centrifuge 7.

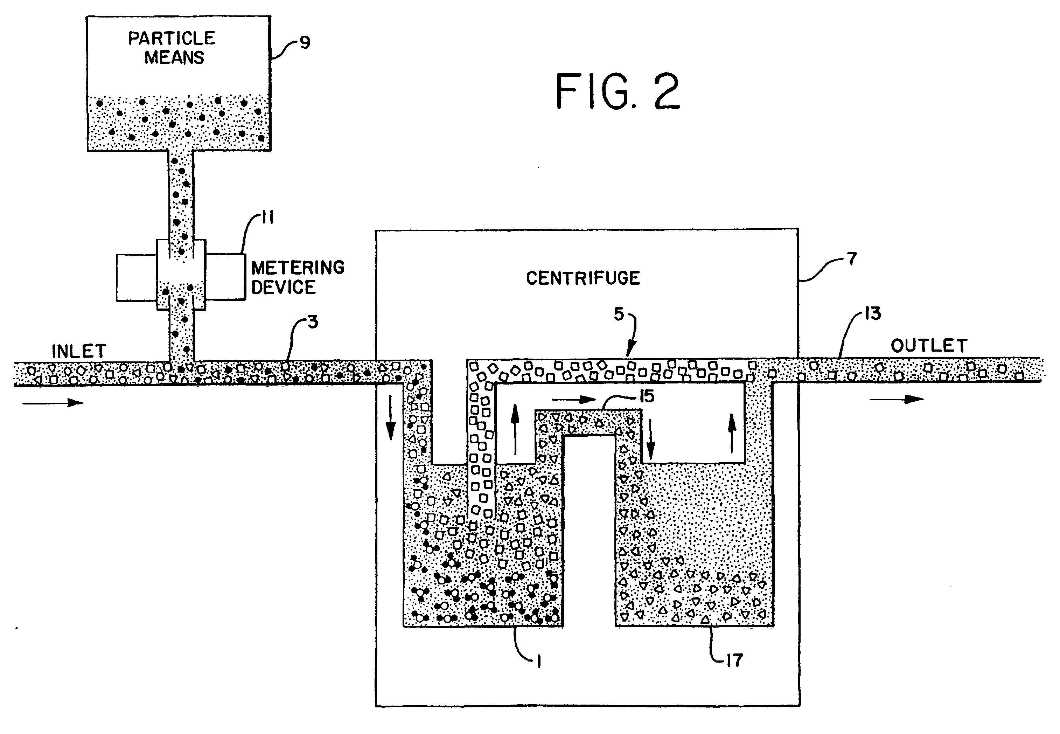

As shown in Figure 2, in this embodiment of the invention first container 1, defining a mixing chamber, is located within centrifuge 7 along with the disposable separation insert 5, rotation of the centrifuge is started, and the particles and blood from the patient are passed aseptically into the first container 1 either as a mixture or as two separate streams. For instance, as illustrated in Figure 2, the particles can be metered from source 9 by means of metering device 11 into a flowing stream of blood in inlet 3. By density centrifugation, the particle/cell complex is captured in container 1 while the remainder of the blood is separated into a lighter component passed by means of outlet 13 out of centrifuge 7 and a heavier component passed by means of conduit 15 into a second container 17 also located within centrifuge 7. The heavier separated component is trapped within second container 17. The lighter component, typically plasma, can be filtered or passed by a magnet to remove residual particles and returned directly to the patient or donor.

A complete, detailed description and illustration of a centrifugal liquid processing system is shown in U.S. Patent No. 4,146,172.

The heterogeneous cell mixtures or concentrates contemplated for use in the practice of this invention are not limited to those derived from whole blood. For instance, a bone marrow preparation can be used in which the cells may be further concentrated and processed in a cell concentrating centrifuge or the like. Additionally, heterogeneous cell mixture can be a tissue-derived cell suspension, or a cell concentrate prepared from peripheral blood using such a centrifugal device. Examples of the latter are concentrates of platelets, lymphocytes, granulocyte, monocytes, or peripheral bone marrow stem cell preparations prepared with a blood cell separator such as the previously described "CS3000 Blood Cell Separator" or the "Autopheresis-C" device.

The beads or particles can be composed of any number of different materials such as polystyrene latex, plastic copolymers, glass, synthetically produced gel beads and the like. Preferably, such materials will possess good mechanical properties to prevent flaking or fracturing of the beads or particles, and will allow chemical covalent attachment with ease.

The beads or particles can contain a paramagnetic particle such as magnetite to allow separation of the bead or particle/cell conjugate using magnets, as described above. For example, particles may be produced in accordance with the methods as described in EP-A-0344270.

Suitable particles for use in the practice of this invention are those that when bound to the target cell will alter the physical properties of the particle/target cell conjugate sufficiently that it can be separated from non-target cells during a continuous flow centrifugation process on the basis of its size, density, magnetic, paramagnetic or electrostatic properties or a combination of two or more of these physical properties. Preferably, the sedimentation velocity and/or density of the particle/cell conjugate is sufficiently altered to enhance continuous flow centrifugal separation of the particle/target cell conjugate from a heterogeneous cell suspension containing other particles of similar density,preferably without the use of a density gradient separation media.

However, if desired, any of a variety of known and commercially available density gradient media, such as "Ficoll-Paque" or "Percoll" might be used to facilitate this separation. "Percoll" is a density gradient consisting of colloidal silica particles (15 - 30mm diameter) coated with non-dialyzable polyvinylpyrrolidone (PVP). "Percoll" gradients can be formed within the density range of 1.0 - 1.3, and are iso-osmatic throughout. "Ficoll-Paque" is an aqueous solution of density gradient $1.077 \pm 0.001$ g/ml, consisting of 5.7 g. "Ficoll 400" and 9 g sodium diatriozate with calcium EDTA per 100 ml. The density and osmolarity are optimized for the isolation of lymphocytes from whole blood.

Further, the particles must either possess an inherent tendency to form a chemical bond with the target cell, or they must be capable of attaching thereto, preferably by means of a covalent bond, a biologic, such as an antibody having an active site that will spontaneously and specifically bond with an antigen on the surface of or attached to the surface of the target cell. The reaction between the particle and the target cell is the basis by which particles exhibit specificity for the target cell population. The reaction involves a receptor and a ligand and can be located at various sites within the inchoate conjugate. Examples of the types of ligand/receptor cell interactions that can be employed are listed below wherein p = particle; polyAB = polyclonal antibody; mAB = monoclonal antibody; PrA = Protein A or Protein G; AG = antigen; AV = avidin; B: = biotinylated; CSR = cell surface receptor; PE = peptide or protein, and / = bond. For instance, P-polyAB/mAB/Ag- target indicates a ligand/receptor binding conjugate consisting of polyclonal antibody bound to a particle that binds a monoclonal antibody that can bind to an antigen present on the target population. In the preferred continuous flow process described below, the particle has already been prepared to bind directly to an antigen, carbohydrate, or cell surface receptor on the target cell. Alternatively, the particle can be prepared to bind to an intermediate in the binding conjugate and the target can be pretreated with the remaining components of the conjugate needed to complete the ligand/receptor bond upon contact. This type of ligand/receptor bonding, when used to separate target cells is called bioselective or immunoaffinity separation.

The methods of immunoaffinity separation using monoclonal antibodies, sheep erythrocyte rosetting, and the like, are well known, and any of the known naturally occurring and synthetically activated affinity separation particles can be employed so long as the particle possesses the capacity to alter the density and/or sedimentation velocity of the target cells. The methods of activating particles for use in affinity separation techniques are also well known. See, for example, U.S. Patent 4,797,475 to Terasaki entitled "Method and Composition for Isolating White Cell Elements" and U.S. Patent 4,415,665 to Mosbach entitled "Method of Covalently Binding Organic Substances to Polymeric Substances". If it is desirable to use magnetic separation techniques to assist in separating the particle/target cell conjugates from other constituents of the suspension or to assist in recovering the particles before the suspension is returned to the patient, the composition of the particles will be selected to include a magnetic or paramagnetic metal. The Dynal Company of Oslos, Norway manufactures paramagnetic microbeads which may be used in accordance with this invention.

As previously stated, the particular biologic materials that can be attached to the particle include antibodies, antigens, proteins avidly bound by cells, glycoproteins, polysaccharides, or lipopolysaccharides. The material may also be a nucleic acid, a lipid molecule, or a synthetic or chemically modified component of such a substance having a selective binding affinity for the cell population to be separated. The methods used for the chemical covalent attachment of biologic materials are known and used in the production of coupled matrix material for affinity chromatography and other selective adsorption applications. Examples of such techniques of covalent attachment to sepharose, gelatine, or other beads may be seen from the following articles: Habeeb, "A Novel Preparation of Immunoadsorbents, "Biochimic et Biophysica Acta, 673 (1981) 527-538; Cambier, et al., "Isolated Phosphorylcholine Binding Lymphocytes. I. Use of a Cleavable Crosslinking Reagent For Solid-Phase Adsorbent Isolation of Functional Antigen Binding Cells," Journal of Immunological Methods, 51 (1982) 209-221; and Bonnafous, et al., "Ligands Immobilized Through Cleavable Mercury-Sulfur Bonds.: Journal of Immunological Methods, 58 (1983) 93-107.

The particles can be suspended in a buffered salt solution, optionally containing a protein such as albumin, selected to be compatible with the physiological requirements of the heterogeneous cell concentrate and the biological binding material attached to the particle means. The chemical properties of the solution can also be selected to confer sterility to the substance covalently attached to the bead or particle. Furthermore, the solution can have chemical properties selected to favor the formation of the bead or particle conjugate when the cell mixture and the particles are combined.

As will be seen from the following examples, the technology is extremely flexible and numerous equivalent alternatives will be apparent to one who is skilled in the arts of immunoaffinity and centrifugal cell separation. Examples of the type of conjugates having one or more ligand/receptor reaction sites that can be employed include, but are not limited to the following:

A. antibody/antigen reactions
P-polyAB/mAB/Ag-target; P-mAB/mAB/Ag-target;
P-mAB/polyAB/Ag-target
P-polyAB/Ag-target; P-mAB/Ag-target

B. Avidin/biotin: antibody/antibody/antigen
P-AV/B:polyAB/mAB/AG-target
P-AV/B:mAB/mAB/Ag-target
P-AV/B:polyAB/Ag-target
P-AV/B:mAB/Ag-target

C. lectin/carbohydrate
P-AV/B:lectin/carbohydrate-target
P-Carbohydrate/Lectin-target
P-Lectin/target

D. Peptide/Cell Surface Receptor (CFR)
P-pep/CSR-target

E. Protein A (or Protein G)/antibody/antigen
P-PrA/polyAB/Ag-target

P-PrA/mAB/Ag-target
P-PrA/polyAB/mAB/Ag-target
P-PrA/mAB/Mab/Ag-target

The linkage between the particle and the target cell formed by the ligand/receptor conjugate must be sufficiently stable to withstand the separation process. Also, the ligand/receptor interaction must be sufficiently specific to provide a high degree of separation of the target cell population from the heterogeneous cell suspension. For positive cell selection, the ligand/receptor conjugate bond can, if necessary, be severed to release the target cell from the particle, but care must be taken to use an appropriate chemical or physical means that does not harm the product.

Suitable particles that can be modified as above described include, but are not limited to, the following list: organic and inorganic materials (such as polymers impregnated with metal, porous silic gel, polystyrene, polyethylene, polypropylene, polyacrylamide, metal, and glass), proteins (such as gelatin or albumin), activated carbohydrates (such as cellulose, agarose, or dextran activated with, for instance, p-toluenesulfonyl chloride or 2, 2, 2, -trifluoroethanesulfonyl chloride) bacteria, and liposomes.

The size of the particles is preferably from about 0.1 to 500, more preferably 0.3 to 80 μm in diameter or effective diameter, if the particles are not symmetrical. The density of the particles is preferably from about 0.25 to 5.0, more preferably 0.5 to 2.5 grams per cubic centimeter. The particles can be added in a liquid suspension to promote efficient particle/cell interaction.

On the order of 10 ml. of such liquid suspension, typically including one hundred thousand to 20 billion particles, may be introduced into the mixing zone, for instance a disposable plastic mixing container that is to receive the cells for separation. If it is undesirable of the particles to remain in the mixing container, for example, due to interaction with the wall of the container, they can be added separately by conventional means such as a sterile connector. Alternatively, the particles can be held within a frangible inner container within the mixing container, as above-described, so that the particles enter the interior of the mixing container when the frangible inner container is broken.

As stated above, the various containers used in this application are preferably integrally linked together in their initial manufacture and are sterilized as a unit to avoid the need for sterile connection during processing in accordance with this invention. However, they may also be connected together with sterile connectors, numerous designs which are well-known, for example, those of U.S. Patent No. Re. 32,056.

The following examples and the other disclosure of this application are provided for illustrative purposes only, and are not intended to limit the scope of the invention, which is as described in the claims below.

EXAMPLE 1

An experiment was conducted to generate a mononuclear cell preparation depleted of a specific leukocyte subpopulation. The cell population selected for depletion was CD4+ lymphocytes. In this experiment, the immunoaffinity particles and blood were incubated together prior to entry into the centrifugal cell separator.

Sheep anti-mouse IgG paramagnetic particles having a density of 1.5 grams per cubic centimeter were purchased from Dynal Inc., located in Oslo, Norway and were coated with anti-CD4 mouse monoclonal antibody (purchased from Becton Dickinson Corp., Mountainview, California) by adding 0.125 ug of antibody per $1x10^7$ beads and incubating overnight at 2-8 degrees Centigrade. ACD anticoagulated whole blood (62 mls. ACD per 450 ml. of blood) was used as the source of CD4+ lymphocytes. Particle/CD4+ lymphocyte complexes were formed by incubating $1x10^{10}$ anti-CD4 Dynal particles and 900 ml. of blood, providing a ratio of 2.2 particles per leukocyte. Incubation was in a closed container at room temperature for 1 hour with end over end rotation at 2.5 rpm.

After the incubation period, the blood/particle mixture was processed using the "Fenwal CS-3000" Blood Cell Separator to generate mononuclear cells. The bag containing the blood/particle mixture was connected to "Fenwal CS-3000" set (Code No.4R2210) via plastic tubing. The volume of the donor blood processed was 0.9 liters. The centrifugal speed was 1,600 rpm and the whole blood flow rate was 60 ml./minute. A granulocyte chamber was used as the separation chamber and a standard Fenwal collection chamber was used to harvest the mononuclear cells. Particle capture was accomplished by density separation within the centrifuge.

The percentage of CD4+ lymphocytes in the unseparated whole blood and in the cells harvested in the collection chamber were determined using a FACSCAN flow cytometer (purchased from Becton Dickinson, Mountainview, CA) after staining the cells with fluorescinated mouse monoclonal anti-CD4 (Becton Dickinson, Mountainview, CA). In the whole blood, 40.3% of the lymphocytes had the CD4 cell surface antigen. The percentage of lymphocytes in the mononuclear cell bag after depletion having the CD4 cell surface antigen was determined to be only 0.4%. Thus, a 2 log reduction in the percentage of CD4+ lymphocytes was accomplished using the immunodensity technique as a batch continuous centrifugation process.

EXAMPLE 2

The same procedure was followed as in Example 1 except that the anti-CD4 beads and the blood were flowing as two separate streams that were joined via a Y connector which led to the separation chamber so that there was no incubation period of the blood and beads prior to entering the centrifuge. The percentage of CD4+ lymphocytes as determined by the FACSCAN flow cytometer was 46.1% in whole blood but only 3.9% in the mononuclear product bag after depletion of CD-4 cells. Thus, the continuous flow immunodensity technique accomplished a 91.5% reduction in the percentage of CD4+ lymphocytes.

EXAMPLE 3

An experiment was conducted in which blood was first fractionated using a Fenwal CS-3000® blood cell separator into a component rich plasma component containing primarily mononuclear cells (MNC) and a platelet component collected in a transfer pack connected to the appropriate effluent line of the separation chamber. Nine hundred mls. of this MNC preparation was used as the source of CD4+ lymphocytes. Anti-CD4 monoclonal antibody coated Dynal particles prepared as described in Example 1 were added to the transfer pack containing the mononuclear cells and then divided into two 445 ml. aliquots. Cell separation was achieved with three processes.

**Process A: Continuous Flow Immunodensity Separation.** One aliquot of bead/cell suspension was pumped into the "CS-3000" collection chamber at a flow rate of 35 ml. per minute. The particle, particle:cell complexes and unbound cells were harvested in the collection chamber. At the end of the run, the contents of the centrifuge collection bag (total volume of 30 ml.) was exposed to a magnetic field to harvest the particles and particle/cell complexes. The cells not captured by the magnetic field were collected and analyzed by flow cytometry.

**Process B: Conventional Rotation Method.** The other aliquot of bead/cell suspension was rotated at 11.4 rpm for 72 minutes at room temperature with 3 ml. samples being collected for flow cytometric analysis after 12, 17, 22, 32, 42 and 72 minutes of incubation. At the end of the 72 minutes of incubation, the remainder of the aliquot was processed as described above with the CS-3000®. The 3ml. aliquots were exposed to a magnetic field to harvest the particles and particle/cell complexes.

**Process C: Rotation Plus Continuous Flow Immunodensity Separation.** The 427 ml. of bead/cell suspension remaining after completion of Process B above were then treated according to the description in Process A above.

In summary, the percentage of CD4+ lymphocytes in each sample was determined using a FACSCAN flow cytometer as described in Example 1. The log depletion of CD4+ lymphocytes achieved for each process was 2.85 for continuous flow immunodensity separation (Process A), and a range from 0.61 - 0.75 after 12 to 75 minutes of incubation for the conventional rotation method (Process B), and 1.89 for the method employing rotation plus continuous flow immunodensity separation (Process C). These data demonstrate the improvement in target cell capture that can be achieved using the invented process as compared to the conventional process of blood separation using magnetic separation alone.

**Claims**

1. A method for separating a specific cell population from a heterogeneous cell mixture comprising:

    intimately contacting a heterogeneous cell mixture with particles comprising binding sites capable of selectively binding to a specific cell population of the cell mixture to form a particle/cell conjugate;
    selectively binding the specific cell population to the particles, creating the particle/cell conjugate;
    separating the particle/cell conjugate from the cell mixture in a chamber (1) of a centrifuge or elutriator (7); and separately collecting the specific cell population;

    **characterised in that**, during binding and separation, said mixture and said particles are introduced continuously into the chamber (1), while mixture, depleted of specific cell population retained in the conjugate, is passed out of the chamber.

2. A method according to Claim 1, wherein said mixture and the particles are passed into the chamber (1) in separate streams.

3. A method according to Claim 1, wherein said mixture and the particles are passed into the chamber (1) as a mixture.

4. A method according to Claim 1, 2 or 3, including the step of separating a selective cell concentrate from a heterogeneous cell mixture and using the concentrate as the cell mixture which is intimately contacted with said particles.

5. The method of any preceding claim, wherein the diameters of the particles are in the range from 0.3 to 80 µm, and the density of the particles is in the range from 0.5 to 2.5 grams per cubic centimeter.

6. The method of Claim 4, wherein the cell concentrate is formed by continuous flow centrifugation.

7. The method of any preceding claim, wherein the cell mixture is selected from whole blood, bone marrow and tissue derived cell suspension.

8. The method of any preceding claim, wherein the specific cell population comprises a nucleated heterogeneous cell population and the ratio of the number of particles to nucleated cell population is from 1:1000 to about 1000:1.

9. The method of Claim 8, wherein the specific cell population comprises a nucleated heterogeneous cell population and the ratio of the number of particles to nucleated cell population is from 1:100 to about 100:1.

10. The method according to Claim 4, wherein the cell concentrate is collected in a collection zone contained within the centrifuge or elutriator (7).

11. The method according to Claim 4, wherein the cell concentrate is collected in a collection zone contained outside of the centrifuge or elutriator (7).

12. The method of any preceding claim, wherein the particles comprise a paramagnetic material and further comprising the step wherein the cell mixture, passing out of the chamber (1), is passed in close proximity to a magnet means for causing any residual particle/cell conjugates and any unbound particles to be retained in a fixed location as remaining, unbound portions of the cell mixture are removed from said fixed location.

13. The method of Claim 1, wherein residual particle/cell conjugates and particles are removed from the cell mixture, passing out of the chamber (1), by a filter.

14. The method of any preceding claim, wherein the binding site is provided by a biologic substance attached to the particles selected from antibodies, antigens, proteins, glycoproteins, polysaccharides, lipopolysaccharides, nucleic acids, and lipids.

15. The method of Claim 4, wherein the cell concentrate comprises a preparation of mononuclear cells separated from bone marrow or neonatal cord blood.

16. The method of any preceding claim in which said particles carry on their surfaces a specific antibody to cells selected from the group consisting of hematopoietic cells, tumor cells, tissue culture cell lines, antigen specific lymphocytes, bacteria, protozoa, virus particles, pathogen infected cells, rDNA transfected cells, plasma proteins, pharmaceuticals, drugs and plant, animal and microbial toxins.

17. The method of Claim 16, wherein the hematopoietic cells are selected from the group consisting of all leukocyte subpopulations and pluripotent stem cells.

18. The method of any preceding claim, wherein the diameter of the particles ranges from 0.1 to 500 μm.

19. The method of any preceding claim, wherein the density of the particles ranges from 0.25 to 5.0 grams per cubic centimeter.

20. An apparatus suitable for use in a method as defined in Claim 1, said apparatus comprising:

    a centrifuge or elutriator (7);
    a source (9) of particles comprising binding sites capable of selectively binding to a specific cell population of the cell mixture to form a particle/cell conjugate and which is located outside of the centrifuge or elutriator (7);
    a first chamber (1) located within the centrifuge or elutriator (7) for receiving the heterogeneous cell mixture and the particles;
    a second chamber (17) for receiving at least a proportion of the heterogeneous cell mixture, depleted of a specific cell population;
    a conduit (15) communicating with the first and second chambers;
    an inlet (3) for aseptically introducing the heterogeneous cell mixture and the particles into the first chamber as a mixture or in separate streams; and
    an outlet (13) from the centrifuge or elutriator (7) through which at least a proportion of the heterogeneous cell mixture depleted of specific cell population can pass.

21. An apparatus according to Claim 20, further comprising a metering device (11) for metering the flow of the particles into the first chamber (1).

22. An apparatus according to Claim 20, wherein the particle source (9) comprises a chamber for mixing the heterogeneous cell mixture and the particles.

23. An apparatus according to Claim 20, wherein the first chamber (1), the inlet (3) and the outlet (13) form a closed, disposable separation insert (5).

24. An apparatus according to Claim 23, further comprising a first apheresis needle attached to the disposable insert (5) for withdrawing whole blood from a patient or donor.

25. An apparatus according to Claim 24, further comprising a second apheresis needle attached to the disposable insert (5) for reinfusing the remainder of the blood into the patient.

**Patentansprüche**

1. Verfahren zum Abtrennen einer spezifischen Zellpopulation von einem heterogenen Zellgemisch, wobei das Verfahren die folgenden Schritte aufweist:

inniges In-Kontakt-Bringen eines heterogenen Zeltgemischs mit Partikeln, die Bindungsstellen aufweisen, die imstande sind, selektiv an eine spezifische Zellpopulation des Zellgemischs zu binden, um ein Partikel-/Zellkonjugat zu bilden;
selektives Binden der spezifischen Zellpopulation an die Partikel unter Schaffung des Partikel-/Zellkonjugats;
Abtrennen des Partikel-/Zellkonjugats von dem Zellgemisch in einer Kammer (1) einer Zentrifuge oder eines Elutriators (7); und separates Sammeln der spezifischen Zellpopulation;

**dadurch gekennzeichnet, daß** das Gemisch und die Partikel während des Bindens und Abtrennens kontinuierlich in die Kammer (1) eingeleitet werden, während gleichzeitig das Gemisch, das hinsichtlich in dem Konjugat zurückgehaltener spezifischer Zellpopulation verarmt ist, aus der Kammer hinaus geleitet wird.

2. Verfahren nach Anspruch 1, wobei das Gemisch und die Partikel in separaten Strömen in die Kammer (1) eingeleitet werden.

3. Verfahren nach Anspruch 1, wobei das Gemisch und die Partikel als ein Gemisch in die Kammer (1) eingeleitet werden.

4. Verfahren nach Anspruch 1, 2 oder 3, das den folgenden Schritt aufweist: Abtrennen eines selektiven Zellkonzentrats von einem heterogenen Zellgemisch und Verwenden des Konzentrats als das Zellgemisch, das mit den Partikel in innigen Kontakt gebracht wird.

5. Verfahren nach einem der vorhergehenden Ansprüche, wobei die Durchmesser der Partikel im Bereich von 0,3 bis 80 μm sind und die Dichte der Partikel im Bereich von 0,5 bis 2,5 g/cm$^3$ ist.

6. Verfahren nach Anspruch 4, wobei das Zellkonzentrat mittels Durchlaufzentrifugation gebildet wird.

7. Verfahren nach einem der vorhergehenden Ansprüche, wobei das Zellgemisch aus von Vollblut, Knochenmark und Gewebe gewonnener Zellsuspension ausgewählt wird.

8. Verfahren nach einem der vorhergehenden Ansprüche, wobei die spezifische Zellpopulation eine kernhaltige heterogene Zellpopulation aufweist und das Verhältnis der Anzahl von Partikeln zu kernhaltiger Zellpopulation 1:1000 bis ungefähr 1000:1 ist.

9. Verfahren nach Anspruch 8, wobei die spezifische Zellpopulation eine kernhaltige heterogene Zellpopulation aufweist und das Verhältnis der Anzahl von. Partikeln zu kernhaltiger Zellpopulation 1:100 bis ungefähr 100:1 ist.

10. Verfahren nach Anspruch 4, wobei das Zellkonzentrat in einer Sammelzone gesammelt wird, die in der Zentrifuge oder dem Elutriator (7) enthalten ist.

11. Verfahren nach Anspruch 4, wobei das Zellkonzentrat in einer Sammelzone gesammelt wird, die außerhalb der Zentrifuge oder des Elutriators (7) enthalten ist.

12. Verfahren nach einem der vorhergehenden Ansprüche, wobei die Partikel ein paramagnetisches Material aufweisen und wobei das Verfahren ferner den Schritt aufweist, bei dem das aus der Kammer (1) austretende Zellgemisch in enger Nachbarschaft zu einer Magneteinrichtung geleitet wird, um zu bewirken, daß eventuelle restliche Partikel-/Zellkonjugate und eventuelle ungebundene Partikel in einer festgelegten Stelle zurückgehalten werden, während verbleibende ungebundene Anteile des Zellgemischs von der festgelegten Stelle entfernt werden.

13. Verfahren nach Anspruch 1, wobei restliche Partikel-/Zellkonjugate und Partikel aus dem aus der Kammer (1) austretenden Zellgemisch durch einen Filter entfernt werden.

14. Verfahren nach einem der vorhergehenden Ansprüche, wobei die Bindungsstelle von einer an den Partikeln anhaftenden biologischen Substanz gebildet wird, die aus Antikörpern, Antigenen, Proteinen, Glykoproteinen, Polysacchariden, Lipopolysacchariden, Nukleinsäuren und Lipiden ausgewählt ist.

15. Verfahren nach Anspruch 4, wobei das Zellkonzentrat ein Präparat aus einkernigen Zellen aufweist, die von Knochenmark oder neonatalem Nabelschnurblut abgetrennt worden sind.

16. Verfahren nach einem der vorhergehenden Ansprüche, wobei die Partikel an ihren Oberflächen einen spezifischen Antikörper für Zellen tragen, die aus der Gruppe ausgewählt werden, die besteht aus hämatopoetischen Zellen, Tumorzellen, Gewebekulturzellinien, antigen-spezifischen Lymphozyten, Bakterien, Protozoa, Viruspartikeln, pathogen infizierten Zellen, rDNA-umgestimmten Zellen, Plasmaproteinen, Pharmazeutika, Arzneistoffen und pflanzlichen, tierischen und mikrobiellen Toxinen.

17. Verfahren nach Anspruch 16, wobei die hamatopoetischen Zellen aus der Gruppe ausgewählt werden, die aus sämtlichen Leukozytensubpopulationen und pluripotenten Stammzellen besteht.

18. Verfahren nach einem der vorhergehenden Ansprüche, wobei der Durchmesser der Partikel im Bereich von 0,1 bis 500 µm liegt.

19. Verfahren nach einem der vorhergehenden Ansprüche, wobei die Dichte der Partikel im Bereich von 0,25 bis 5,0 g/cm$^3$ liegt.

20. Vorrichtung, die zum Gebrauch in einem Verfahren nach Anspruch 1 geeignet ist, wobei die Vorrichtung folgendes aufweist:

eine Zentrifuge oder einen Elutriator (7);
einen Vorrat (9) an Partikeln, die Bindungsstellen aufweisen, die imstande sind, selektiv an eine spezifische Zellpopulation des Zellgemischs zu binden, um ein Partikel-/Zellkonjugat zu bilden, wobei der Vorrat außerhalb der Zentrifuge oder des Elutriators (7) angeordnet ist;
eine erste Kammer (1), die innerhalb der Zentrifuge oder des Elutriators (7) angeordnet ist, zur Aufnahme des heterogenen Zellgemischs und der Partikel;
eine zweite Kammer (17) zur Aufnahme von mindestens einem Anteil des heterogenen Zellgemischs, das hinsichtlich einer spezifischen Zellpopulation verarmt ist;
eine Leitung (15), die mit der ersten und der zweiten Kammer in Verbindung steht;
einen Einlaß (3) zum aseptischen Einleiten des heterogenen Zellgemischs und der Partikel in den ersten Behälter als ein Gemisch oder in separaten Strömen; und
einen Auslaß (13) aus der Zentrifuge oder dem Elutriator (7), durch den mindestens ein Anteil des heterogenen Zellgemisch hindurchgehen kann, das hinsichtlich der spezifischen Zellpopulation verarmt ist.

21. Vorrichtung nach Anspruch 20, die ferner eine Dosiereinrichtung (11) zum Dosieren des Durchflusses der Partikel in den ersten Behälter (1) aufweist.

22. Vorrichtung nach Anspruch 20, wobei der Partikelvorrat (9) eine Kammer zum Vermischen des heterogenen Zellgemischs und der Partikel aufweist.

23. Vorrichtung nach Anspruch 20, wobei die erste Kammer (1), der Einlaß (3) und der Auslaß (13) einen geschlossenen Einmal-Trenneinsatz (5) bilden.

24. Vorrichtung nach Anspruch 23, die ferner eine an dem Einmaleinsatz (5) angebrachte erste Apheresenadel zur Entnahme von Vollblut von einem Patienten oder Spender aufweist.

25. Vorrichtung nach Anspruch 24, die ferner eine an dem Einmaleinsatz (5) angebrachte zweite Apheresenadel zum Reinfundieren des restlichen Bluts in den Patienten aufweist.

## Revendications

1. Procédé pour séparer une population de cellules spécifiques d'un mélange de cellules hétérogènes, ledit procédé, qui comprend :

le contact intime d'un mélange de cellules hétérogènes avec des particules comprenant des sites de liaison capables de fixer sélectivement une population de cellules spécifiques pour former un conjugué particule/cellule ;
la fixation sélective de la population de cellules spécifiques sur les particules, qui crée le conjugué particule/cellule ;
la séparation du conjugué particule/cellule du mélange de cellules dans une chambre (1) d'une centrifugeuse ou d'un séparateur par décantation (7) ; et le recueil séparément de la population de cellules spécifiques ;
étant **caractérisé en ce que**, durant la fixation et la séparation, ledit mélange et lesdites particules sont introduits en continu dans la chambre (1), tandis que le mélange, appauvri en la population de cellules spécifiques retenue dans le conjugué, est sorti de la chambre.

2. Procédé suivant la revendication 1, dans lequel ledit mélange et lesdites particules sont introduits dans la chambre (1) selon des courants séparés.

3. Procédé suivant la revendication 1, dans lequel ledit mélange et lesdites particules sont introduits dans la chambre (1) sous la forme d'un mélange.

4. Procédé suivant la revendication 1, 2 ou 3, comprenant l'étape de séparation d'un concentré cellulaire sélectif d'un mélange de cellules hétérogènes et d'utilisation du concentré en tant que mélange cellulaire qui est intimement mis en contact avec lesdites particules.

5. Procédé suivant l'une quelconque des revendications précédentes, dans lequel les diamètres des particules se situent dans l'intervalle de 0,3 à 80 µm, et la densité des particules se situe dans l'intervalle de 0,5 à 2,5 g/cm³.

**6.** Procédé suivant la revendication 4, dans lequel le concentré cellulaire est formé par centrifugation en continu (en anglais: "continuous flow centrifugation").

**7.** Procédé suivant l'une quelconque des revendications précédentes, dans lequel le mélange cellulaire est choisi parmi le sang total, la moelle osseuse et une suspension de cellules provenant d'un tissu.

**8.** Procédé suivant l'une quelconque des revendications précédentes, dans lequel la population de cellules spécifiques comprend une population de cellules hétérogènes nucléées, le rapport du nombre de particules à la population de cellules nucléées étant d'environ 1:1000 à 1000:1.

**9.** Procédé suivant la revendication 8, dans lequel la population de cellules spécifiques comprend une population de cellules hétérogènes nucléées, le rapport du nombre de particules à la population de cellules nucléées étant d'environ 1:100 à 100:1.

**10.** Procédé suivant la revendication 4, dans lequel le concentré cellulaire est recueilli dans une zone de recueil située dans la centrifugeuse ou le séparateur par décantation (7).

**11.** Procédé suivant la revendication 4, dans lequel le concentré cellulaire est recueilli dans une zone de recueil située à l'extérieur de la centrifugeuse ou du séparateur par décantation (7).

**12.** Procédé suivant l'une quelconque des revendications précédentes, dans lequel les particules comprennent un matériau paramagnétique, et qui comprend en outre l'étape selon laquelle le mélange cellulaire sortant de la chambre (1) passe à proximité étroite d'un moyen d'aimantation (en anglais: "magnet means") de façon à retenir au niveau d'une position déterminée les conjugués particule/cellule résiduels et les particules non liées, tandis que les portions restantes non liées du mélange cellulaire sont séparées de ladite position déterminée.

**13.** Procédé suivant la revendication 1, dans lequel les conjugués particule/cellule et les particules sont séparés du mélange cellulaire, qui sort de la chambre (1), par un filtre.

**14.** Procédé suivant l'une quelconque des revendications précédentes, dans lequel le site de liaison est fourni par une substance biologique attachée aux particules et choisie parmi les anticorps, les antigènes, les protéines, les glycoprotéines, les polysaccharides, les lipopolysaccharides, les acides nucléiques et les lipides.

**15.** Procédé suivant la revendication 4, dans lequel le concentré cellulaire comprend une préparation de cellules mononucléaires séparées de la moelle osseuse ou du sang de cordon néonatal.

**16.** Procédé suivant l'une quelconque des revendications précédentes, dans lequel lesdites particules portent sur leurs surfaces un anticorps spécifique de cellules appartenant à l'ensemble constitué par les cellules hématopoïétiques, les cellules tumorales, les lignées cellulaires de culture de tissu, les lymphocytes spécifiques d'antigène, les bactéries, les protozoaires, les particules virales, les cellules infectées par un pathogène, les cellules transfectées par rDNA, les protéines plasmatiques, les produits pharmaceutiques, les médicaments et les toxines plantaires, animales et microbiennes.

**17.** Procédé suivant la revendication 16, dans lequel les cellules hématopoïétiques sont choisies parmi l'ensemble constitué par toutes les sous-populations leucocytaires et les cellules tigées pluripotentes (en anglais : pluripotent stem cells).

**18.** Procédé suivant l'une quelconque des revendications précédentes, dans lequel le diamètre des particules se situe dans l'intervalle de 0,1 à 500 μm.

**19.** Procédé suivant l'une quelconque des revendications précédentes, dans lequel la densité des particules se situe dans l'intervalle de 0,25 à 5,0 g/cm³.

**20.** Appareillage pouvant convenir pour utilisation dans le procédé de la revendication 1, ledit dispositif comprenant :

une centrifugeuse ou un séparateur par décantation (7) ;
une source (9) de particules comprenant des sites de liaison capables de fixer sélectivement une population de cellules spécifiques d'un mélange de cellules pour former un conjugué particule/cellule et qui est située à l'extérieur de la centrifugeuse ou du séparateur par décantation (7) ;
une première chambre (1) située à l'intérieur de la centrifugeuse ou du séparateur par décantation (7) pour recevoir le mélange de cellules hétérogènes et les particules ;
une seconde chambre (17) pour recevoir au moins une proportion du mélange de cellules hétérogènes appauvri en une population de cellules spécifiques ;
un conduit (15) communiquant avec les première et seconde chambres ;
une entrée (3) pour introduire aseptiquement le mélange de cellules hétérogènes et les particules dans un premier réservoir, sous la forme d'un seul mélange ou sous la forme de deux courants séparés ; et

une sortie (13) de la centrifugeuse ou du séparateur par décantation (7) par laquelle peut passer au moins une proportion du mélange de cellules hétérogènes appauvri en population de cellules spécifiques.

21. Appareillage suivant la revendication 20, comprenant en outre un dispositif de mesure (11) pour mesurer le courant de particules entrant dans le premier réservoir (1).

22. Appareillage suivant la revendication 20, dans lequel la source (9) de particules comprend une chambre pour mélanger le mélange de cellules hétérogènes et les particules.

23. Appareillage suivant la revendication 20, dans lequel ladite chambre (1), l'entrée (3) et la sortie (13) forment un insert de séparation fermé, pouvant être enlevé (5).

24. Appareillage suivant la revendication 23, comprenant en outre une première aiguille d'aphérèse (en anglais : "apheresis needle") attachée à l'insert pouvant être enlevé (5) pour soutirer le sang total d'un patient ou d'un donneur.

25. Appareillage suivant la revendication 24, comprenant en outre une seconde aiguille d'aphérèse attachée à l'insert pouvant être enlevé (5) pour réinjecter le reste du sang au patient.

FIG. I

MONONUCLEAR CELL PREP /WITH PARTICLE MEANS

10

CENTRIFUGE

5

7

3

INLET

OUTLET

EP 0 653 062 B1

FIG. 2

PARTICLE MEANS 9

METERING DEVICE 11

CENTRIFUGE 7

INLET 3

OUTLET 13

5

15

1

17

EP 0 653 062 B1